# EUROPEAN PATENT APPLICATION

(11) **EP 3 659 498 A2**
(43) Date of publication of application: **03.06.2020**
(21) Application number: 19211931.1
(22) Date of filing: 27.11.2019
(51) Int. Cl.: A61B 5/00, C12Q 1/6876

(54) **SYSTEMS FOR PROVIDING AN EARLY INDICATOR OF TRUE POSITIVE PROBABILITY OF A MICROBIOLOGY TEST SAMPLE TO IMPROVE TREATMENT RESPONSE TIME AND ACCURACY**

(30) Priority: 27.11.2018 US 201862771693 P
(71) Applicant: Stone Medical Corporation, Bettendorf, IA 52722 (US); Stone, Benjamin Curtis, Davenport, IA 52804 (US)
(72) Inventor: Stone, Benjamin Curtis, Davenport, IA Iowa 52804 (US)
(74) Representative: Stevenson-Hill, Jack Patrick

(57) **Abstract**

The present invention is a system for tracking microbiology test samples with relatively lower rates of contamination in order to separate test results from samples with a higher positive predictive value (PPV) from those with a lower PPV in order to provide an early indicator of true positive probability and prevent delays in appropriate treatment of patients.

## Description

### CROSS REFERENCE TO RELATED APPLICATIONS

N/A

### FIELD OF INVENTION

This present disclosure relates to systems for providing an early indicator of true positive probability of a microbiology test sample to improve treatment response time and accuracy.

### STATEMENT REGARDING FEDERALLY SPONSORED RESEARCH OR DEVELOPMENT

No federal funds were used to develop or create the invention disclosed and described in the patent application.

### REFERENCE TO SEQUENCE LISTING, A TABLE, OR A COMPUTER PROGRAM LISTING COMPACT DISK APPENDIX

Not Applicable

### BACKGROUND

Frequent contamination of microbiology test samples often limits the usefulness of the test by reducing the positive predictive value (PPV) below the point of reliability. It is not uncommon for body fluid cultures on blood, urine, and other samples to have contamination rates (false positive rates) that exceed the true positive rates. This lack of reliability can lead to significant delays in general treatment of patients with broad spectrum antibiotics, failure to perform antimicrobial sensitivity testing on positive samples, and an even greater delay in treatment of patients with more targeted narrow spectrum antibiotics. Delayed general treatment can increase the mortality rate in cases of sepsis, septicemia, and other rapidly progressing diseases. Delays in delivery of targeted treatment can expose the patient to longer general treatment with potentially severe side effects such as *Clostridium difficile* infections, vancomycin resistant *Enterococcus* infections, and damage to the liver and/or kidneys.

Microbiology tests such as blood cultures, urine cultures, and other body fluid cultures are often performed in multiple sets to help determine if a test result is a contaminated false positive or a true positive finding. Contamination is typically indicated when one test result is positive and several other test results from the same patient are negative. However, this determination is retrospective, often requiring several days and most of the test results before the sample can be identified as contaminated. This type of protocol has limited value towards treatment decisions in the earlier stages of sepsis when general or targeted treatment may have the greatest impact on patient outcomes.

U.S. Patent 6,913,580 discloses various methods and systems to reduce contamination of body fluid samples by diverting the initial body fluid flow prior to collection of a body fluid culture sample. While these methods are effective at reducing contamination and yield a high PPV, they may represent only a portion of the test results if compliance with usage is low or moderate. The ability to track and report test results from body fluid samples drawn with these types of methods along with the PPV, contamination rate, or another indicator of true positive probability can provide an early indicator that prevents delays in treatment of patients. The reduced time to general and targeted treatment can reduce rates of mortality, morbidity, and improve outcomes.

### SUMMARY OF THE PRESENT DISCLOSURE

The present disclosure describes systems for tracking microbiology test samples with relatively lower rates of contamination in order to separate test results from samples with a higher positive predictive value (PPV) from those with a lower PPV (wherein the PPV may be dependent at least in part on the system and/or method used to procure the body fluid sample). The PPV of a diagnostic test may be essential to healthcare providers when interpreting the results of the test. For example, a relatively high PPV can support decisions to treat patients without significant delay, while knowledge of a low PPV may lead healthcare workers to reconsider a patient's complete clinical case prior to providing treatment that may not be necessary, and/or may require retesting. This aspect of the present disclosure may include a system and/or method for separating diagnostic test results into groups based on the system and/or method of procuring the body fluid sample that was used for the diagnostic test, determining the PPV for those different groups, and reporting the PPV of the corresponding group to healthcare workers along with the diagnostic test results for a given patient. This system can prevent delay of appropriate treatment and simultaneously reduce the frequency of unnecessary treatment.

In more detail, according to an aspect of the invention there is provided a system for providing an early indicator of true positive probability of a microbiology test sample to improve treatment response time and accuracy comprising the steps of: providing a means to collect a first body fluid sample comprising a first seal having a contact area and a first hollow body; providing a means to maintain sterility of the contact area; providing a means to identify a microbiology test sample; providing a means to collect a microbiology test sample comprising a second seal and a second hollow body; providing a means to access a patient's body fluid vessel comprising a first end and a second end; drawing a microbiology test sample using a collection method comprising the steps of collecting a body fluid sample by piercing through a patient's skin using said first end of said means to access a patient's body fluid vessel and piercing through said first seal using said second end of said means for accessing a patient's body fluid vessel such that potential contaminants are drawn into said means for collecting a first body fluid sample, collecting a microbiology test sample by piercing though said second seal of said means to collect a microbiology test sample using said second end of said means to access a patient's body fluid vessel; performing a microbiology test using said microbiology test sample to yield a test result; using said means to identify a microbiology test sample to classify said test result into a group of test results; calculating a contamination rate for said group of test results; generating an indicator of true positive probability based on said contamination rate; generating a report comprising said test result and said indicator of true positive probability.
said means for identifying a microbiology test sample may be attached to said means for collecting a first body fluid sample.

Said means for identifying a microbiology test sample may be attached to said means for collecting a microbiology test sample.

Said means for identifying a microbiology test sample may be a scannable identifier.

Said means for identifying a microbiology test sample may be a needle holder having a marking device that may be coupled to said means to access a patient's body fluid vessel.

Said microbiology test may be a blood culture test.

Said microbiology test may be a polymerase chain reaction test.

Said indicator of contamination probability may be a positive predictive value.

According to another aspect of the invention there is provided a system for providing an early indicator of true positive probability of a microbiology test sample to improve treatment response time and accuracy comprising the steps of: collecting a microbiology test sample into a device using a collection method; attaching a scannable identifier onto said device; performing a microbiology test using said microbiology test sample to yield a microbiology test result; entering a microbiology test result data point that corresponds to said microbiology test result into an electronic recordkeeping system; entering an identifier data point indicating presence of said scannable identifier on said device into said electronic recordkeeping system; associating said identifier data point with said microbiology test result data point within said electronic recordkeeping system; calculating a contamination rate for a group of test results collected using said collection method; generating an indicator of true positive probability based on said contamination rate; generating an indicator of true positive probability data point corresponding to said indicator of true positive probability; generating a report comprising said microbiology test result data point and said indicator of true positive probability data point.

Said scannable identifier may be a bar code.

Said scannable identifier may be a radiofrequency tag.

Said device may be a blood culture bottle.

Said indicator of true positive probability may be a positive predictive value.

Any of the optional features discussed above in relation to one aspect of the invention may, where appropriate, be applied to another aspect of the invention.

### BRIEF DESCRIPTION OF THE FIGURES

FIG. 1 is a flow chart illustrating a first preferred embodiment of the system for providing an early indicator of true positive probability of a microbiology test sample to improve treatment response time and accuracy.
FIG. 2 is a flow chart illustrating a second preferred embodiment of the system for providing an early indicator of true positive probability of a microbiology test sample to improve treatment response time and accuracy.
FIG. 3 is an illustrative depiction of a blood collection tube with a contact area and a tube seal cap.
FIG. 4 is an illustrative depiction of the blood collection tube with the tube seal cap in place to maintain sterility of the contact area.
FIG. 5 is an illustrative depiction of a needle holder having a marking device for tracking microbiology test samples.
FIG. 6 is an illustrative depiction of the needle holder engaged with a device for collecting a microbiology test sample such that the marking device is in contact with the device for collecting a microbiology test sample.

### DETAILED DESCRIPTION OF THE PREFERRED EMBODIMENTS

Preferred embodiments of a system for providing an early indicator of true positive probability of a microbiology test sample to improve treatment response time and accuracy are described here comprising the steps of: providing a means to collect a first body fluid sample comprising a first seal having a contact area and a first hollow body; providing a means to maintain sterility of the contact area; providing a means to identify a microbiology test sample; providing a means to collect a microbiology test sample comprising a second seal and a second hollow body; providing a means to access a patient's body fluid vessel comprising a first end and a second end; drawing a microbiology test sample using a collection method comprising the steps of collecting a body fluid sample by piercing through a patient's skin using the first end of the means to access a patient's body fluid vessel and piercing through the first seal using the second end of the means for accessing a patient's body fluid vessel such that potential contaminants are drawn into the means for collecting a first body fluid sample, collecting a microbiology test sample by piercing though the second seal of the means to collect a microbiology test sample using the second end of the means to access a patient's body fluid vessel; performing a microbiology test using the microbiology test sample to yield a test result; using the means to identify a microbiology test sample to classify the test result into a group of test results; calculating a contamination rate for the group of test results; generating an indicator of true positive probability based on the contamination rate; generating a report comprising the test result and the indicator of true positive probability.

A first preferred embodiment of a system for providing an early indicator of true positive probability of a microbiology test sample to improve treatment response time and accuracy is described that may be preferable for ease of initial setup and cost efficiency.

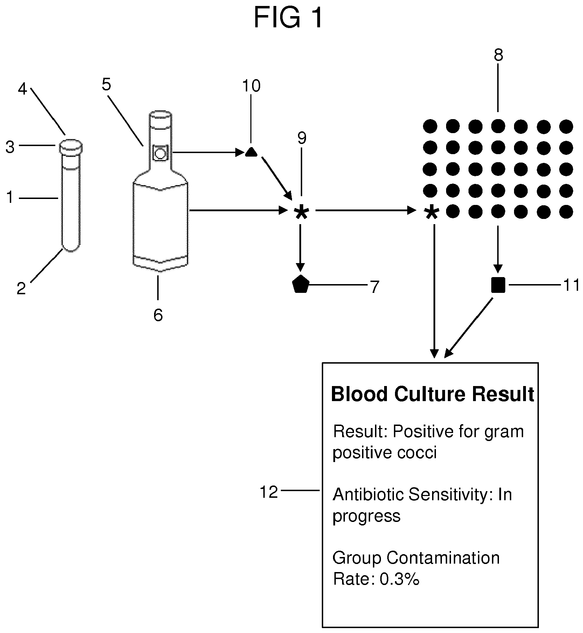

Referring to FIG 1, a first preferred embodiment of the means to collect a first body fluid specimen is a blood collection tube 1. The blood collection tube 1 may be, but is not limited to, an evacuated specimen tube comprising a hollow body 2, a seal 3 closing said hollow body 2, and a relatively lower interior pressure allowing for inflow of a body fluid sample when the seal 3 is pierced by a needle. The seal has a contact area 4 with an exterior facing aspect that could be subjected to environmental exposure and potential contaminants. A variety of blood collection tubes may be used and this disclosure should not be construed as to limit the blood collection tube to those described.

A first preferred embodiment of the means to maintain sterility of the contact area 4 is a sealed package. The sealed package may be constructed from polypropylene, medical grade paper, or other packing materials well known in the art. The sealed package may be hermitically sealed by heating, adhesives, or other sealing processes well known in the art. The sealed package may encompass only the blood collection tube 1, or the blood collection tube 1 and other materials for collecting a microbiology test specimen.

Referring to FIG 1, the first preferred embodiment of the means to identify a microbiology test sample is a decal 5. The decal 5 is of the kind comprising an adhesive capable of securing the decal 5 to a surface. The decal may be attached to a non-adhesive backing material.

A first preferred embodiment of a kit for collecting a blood culture sample is the sealed package configured to envelop the blood collection tube 1 and decal 5. The kit for collecting a blood culture sample may further comprise a variety of additional materials such as a needle hub, an IV extension, a blood culture bottle adaptor, an antiseptic, a set of gloves, a tourniquet, an IV dressing, a bandage, a blood culture bottle, or the blood collection needle. A variety of designs and components are suitable for the kit for collecting a blood culture sample and this disclosure should not be construed as to limit the amount or type of additional materials. The kit for collecting a blood culture sample may also be sterilized partially or entirely using ethylene oxide, gamma irradiation, or other sterilization procedures well known in the art.

Referring to FIG 1, a first preferred embodiment of the means to collect a microbiology test sample is a blood culture bottle 6 comprising a second hollow body, a second seal, and a liquid medium. The second hollow body may be formed from glass or thermoplastic materials. The second seal may be formed from a synthetic elastomer or natural rubber. The liquid medium may be a trypticase soy broth or other medium that supports the growth of microorganisms. A variety of materials are suitable for the blood culture bottle 6 and this disclosure should not be construed as to limit types of materials used to construct the blood culture bottle 6.

A first preferred embodiment of the means to access a patient's body fluid vessel is a blood collection needle having a first end and a second end. The first end may be beveled for easier insertion through the patient's skin and into the vein. The second end may be beveled for easier passage through elastomeric seals. The blood collection needle may be constructed from steel and thermoplastic. A variety of materials are suitable for the blood collection needle and this disclosure should not be construed as to limit types of materials used to construct the blood collection needle. The blood collection needle may further comprise a hollow tube disposed between the first end and the second end or a safety shield that can be configured to enclose the first end.

A first preferred embodiment of the collection method uses the blood collection tube 1 to remove potential contaminants from the blood collection needle prior to collection of the blood culture sample into the blood culture bottle 6. The collection method may further comprise a step of collecting a second blood culture bottle. After collection of the blood culture sample into the blood culture bottle, the decal 5 is attached to the surface of the blood culture bottle 6.

A first preferred embodiment of the microbiology test is a blood culture test comprising the steps of placing the blood culture bottle 6 into an incubator and monitoring the blood culture bottle 8 for a signal of microbial growth yielding a blood culture test result.

The blood culture test result may be a positive blood culture test result or a negative blood culture test result for microbial growth. Following a positive blood culture test result, the blood culture test further comprises the steps of removing a subculture sample from the blood culture bottle 6, performing a plate-based subculture test on the subculture sample, and performing an antimicrobial sensitivity test 7 on the subculture sample. Alternatively, the blood culture test may further comprise a test using polymerase chain reaction. A negative test result may be determined after a predetermined period of time in which the signal of microbial growth is not observed.

Referring to FIG 1, the blood culture test result is classified into a group of blood culture test results 8 yielded from blood culture tests performed on samples drawn using the same general steps described for the first embodiment of the collection method. Classification of the blood culture test result is performed using a first classification procedure comprising the steps of: entering a blood culture test result data point 9 corresponding to the blood culture test result into a recordkeeping file; entering a decal data point 10 indicating observation of the decal 5 on the blood culture bottle 6 into the recordkeeping file; associating the blood culture test result data point 9 and the decal data point 10.

The steps of the first classification procedure may be performed in a different sequence and this disclosure should not be construed as to limit the first classification procedure to the order described.

A first preferred embodiment of the indicator of true positive probability is a blood culture contamination rate. The blood culture contamination rate is calculated using a first analysis procedure comprising the steps of: counting the number of blood culture test results from the group of blood culture test results 8 to yield a total number of blood culture test results; establishing a first criteria for identifying a false positive blood culture test result that indicates a contaminated blood culture sample; applying the first criteria to the group of blood culture test results 8; counting the number of false positive blood culture test results; dividing the number of false positive blood culture test results by the total number of blood culture test results to yield the blood culture contamination rate for the group of blood culture test results; generating a blood culture contamination rate data point 11 corresponding to the blood culture contamination rate; entering the contamination rate data point 11 into the recordkeeping file.

The first analysis procedure may be performed by a manual calculation, electronic calculation, or a combination of manual and electronic methods. The steps of the first analysis procedure may be performed in a different sequence and this disclosure should not be construed as to limit the first analysis procedure to the order described.

Referring to FIG 1, a first preferred embodiment of the report is a blood culture report 12. The blood culture report 12 is generated using a first reporting procedure comprising the steps of: displaying the blood culture test data point 9 on the report 12; associating the decal data point 10 to the blood culture test result data point 9; displaying the blood culture contamination rate data point 11 on the report 12.

The steps of the first reporting procedure may be performed in a different sequence and this disclosure should not be construed as to limit the first reporting procedure to the order described.

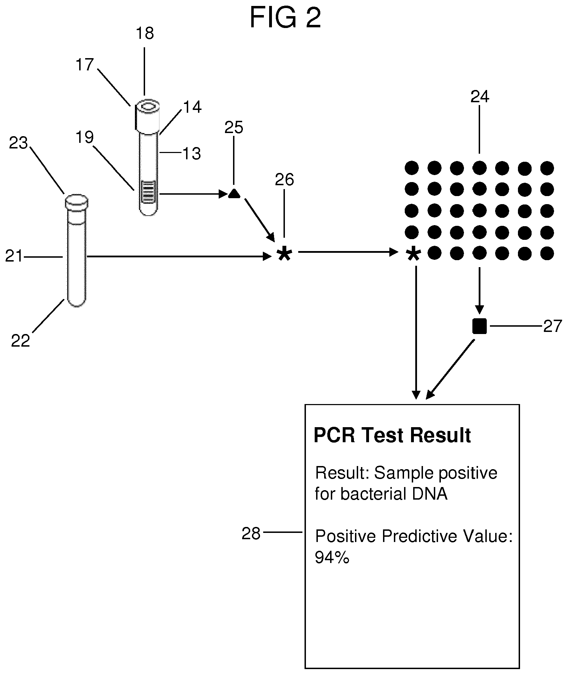

Referring to FIG 2, a second preferred embodiment of the system for providing an early indicator of true positive probability of a microbiology test sample to improve treatment response time and accuracy is described using more advanced materials and techniques. The second preferred embodiment may be preferable to achieve greater system consistency.

Referring to FIG 2, a second preferred embodiment of the means to collect a first body fluid specimen is a pre-marked blood collection tube 13 comprising a hollow body 14, a seal 15 closing said hollow body 14, a contact area 16, a tube cap 17 having an access window 18, and a scannable identifier 19 attached to the hollow body 14. The scannable identifier 19 may be a line type barcode, a matrix barcode, a radiofrequency tag, or another type of scannable material or device. A variety of materials may be used for the scannable identifier 19 and this disclosure should not be construed as to limit the scannable identifier to those described. The scannable identifier 19 may further comprise an adhesive for attachment to the hollow body 14.

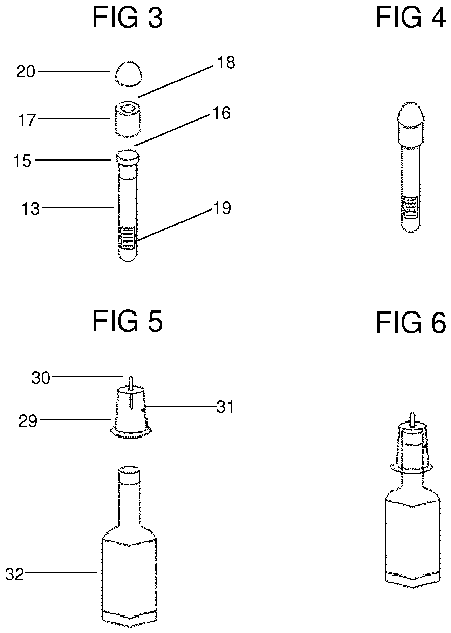

Referring to FIG 3, a second preferred embodiment of the means to maintain sterility of the contact area 16 is a tube cap seal 20. As shown in FIG 4, the tube cap seal 20 is situated over the contact area 16 prior to or after sterilization in order to maintain sterility of the contact area 16. The tube cap seal 20 depicted in FIG 3 is reversibly attached to the access window 18 in the tube cap 17. In other embodiments, a tube cap seal may be reversibly attached to the hollow body 14 or to the seal 15. The tube cap seal may be attached by adhesive, screw threading, friction, or other means of attachment. The tube cap seal 20 may be constructed from injection molded thermoplastic, extruded thermoplastic film, or other materials. A variety of configurations are suitable for the tube cap seal 20 and this disclosure should not be construed as to limit configuration or design of the tube cap seal 20 to those described.

The second preferred embodiment of the means to collect a microbiology test sample is a nucleic acid preservation tube 21 comprising a hollow body 22, a seal 23, and a preservative for nucleic acids. The hollow body 22 can be formed from glass or thermoplastic materials. The seal 23 may be formed from a synthetic elastomer or natural rubber. The preservative may comprise an anticoagulant such as ethylenediamine tetraacetic acid or sodium citrate, a nucleic acid stabilizing agent, or a combination of additives and solutions. A variety of preservatives and additives are suitable for the nucleic acid preservation tube and this disclosure should not be construed as to limit the types of preservatives and additive used to those described.

A second preferred embodiment of the means to access a patient's body fluid vessel is an intravenous needle assembly comprising an intravenous needle comprising cylindrical body and a first end, an intravenous catheter comprising a first female luer fitting and a hollow body, and an access device comprising a first male luer fitting and a second end. The intravenous device assembly is configured such that the cylindrical body is at least partially situated inside the intravenous catheter prior to insertion into the patient's body fluid vessel. The first end may be beveled for easier insertion through the patient's skin and into the vein. The second end may be beveled for easier passage through elastomeric seals. The intravenous needle may be constructed from steel and thermoplastic. The intravenous catheter may be constructed from thermoplastic. The access device may be constructed from thermoplastic and stainless steel. A variety of materials are suitable for construction of the intravenous needle assembly and this disclosure should not be construed as to the limit the materials to those described. The intravenous needle assembly may further comprise an extension device comprising a second male luer fitting, a second female luer fitting, and a hollow extension tube disposed between the second male luer fitting and the second female luer fitting.

A second preferred embodiment of the collection method uses the pre-marked blood collection tube 13 to remove potential contaminants from the intravenous catheter prior to collection of the microbiology test sample into the nucleic acid preservation tube and comprises the steps of: collecting a body fluid sample by piercing through a patient's skin using the first end of the intravenous needle assembly; removing the intravenous needle from the intravenous catheter such that a portion of the hollow body of the intravenous catheter remains in a patient's body fluid vessel; attaching the first male luer fitting of the access device to the first female luer fitting of the intravenous catheter; piercing through the seal 15 of the pre-marked blood collection tube 13 using the second end of the access device such that potential contaminants are drawn into the pre-marked blood collection tube 13; collecting a microbiology test sample by piercing though the seal 23 of the nucleic acid preservation tube using the second end of the access device.

A second preferred embodiment of the microbiology test is a polymerase chain reaction test comprising the steps of: addition of a lysis reagent to the microbiology test sample; addition of a deoxyribonucleic acid degradation agent to the microbiology test sample; centrifugation of the microbiology test sample and removal of a supernatant; addition of a cell wall lysis reagent to the microbiology test sample; transfer of the microbiology test sample to a purification column; addition of a washing solution to the purification column, addition of an eluting solution to the microbiology test sample; addition of a mixture comprising a primer set specific for microorganism related nucleic acid polymer sequences, a polymerase, and a plurality of nucleotides to the microbiology test sample; placement of the microbiology test sample in a polymerase chain reaction chamber; cycling the microbiology test sample through a denaturing temperature phase, an annealing temperature phase, and an elongation temperature phase; addition of a reporter to the microbiology test sample; observation of the microbiology test sample for the reporter to yield a polymerase chain reaction test result.

The polymerase chain reaction test result may be a positive test result or a negative test result for the reporter indicating the presence of microorganism-related nucleic acid polymer sequences. A variety of materials, reagents, solutions, and steps are suitable for the polymerase chain reaction test and this disclosure should not be construed as to the limit the materials, reagents, solutions, and steps to those described.

Referring to FIG 2, the polymerase chain reaction test result is classified into a group of polymerase chain reaction test results 24 yielded from polymerase chain reaction tests performed on body fluid samples drawn using the same general steps described for the second embodiment of the collection method. Classification of the polymerase chain reaction test result is performed using a second classification procedure comprising the steps of: scanning the scannable identifier 19 using a scanner; generating an identifier data point 25 indicating presence of the pre-marked blood collection tube 13 and nucleic acid preservation tube 21; entering a polymerase chain reaction test result data point 26 corresponding to the polymerase chain reaction test result into a computer based electronic recordkeeping system; associating the identifier data point 25 and the polymerase chain reaction test result data point 26.

The steps of the second classification procedure may be performed in a different sequence and this disclosure should not be construed as to limit the second classification procedure to the order described. Scanning of the scannable identifier 19 may occur during receiving of the pre-marked blood collection tube 13 and prior to the polymerase chain reaction test.

The second preferred embodiment of the indicator of true positive probability is a positive predictive value. The positive predictive value is calculated using a second analysis procedure comprising the steps of: counting the number polymerase chain reaction test results from the group of polymerase chain reaction test results 24 to yield a total number of polymerase chain reaction test results; counting the number of positive test results from the group of polymerase chain reaction test results 24; establishing a second criteria for identifying a false positive polymerase chain reaction test result that indicates a contaminated body fluid sample; applying the second criteria to the group of polymerase chain reaction test results 24; counting the number of false positive polymerase chain reaction test results; dividing the number of false positive polymerase chain reaction test results by the number of positive test results to yield the positive predictive value for the group of polymerase chain reaction test results; generating a positive predictive value data point 27 corresponding to the positive predictive value.

The second analysis procedure may be performed by a manual calculation, the computer based electronic software, or a combination of methods. The steps of the first analysis procedure may be performed in a different sequence and this disclosure should not be construed as to limit the second analysis procedure to the order described.

Referring to FIG 2, a second preferred embodiment of the report is a polymerase chain reaction test report 28. The polymerase chain reaction test report 28 is generated using a second reporting procedure comprising the steps of: displaying the polymerase chain reaction test data point 25 on the polymerase chain reaction test report 28; associating the identifier data point 25 to the polymerase chain reaction test result data point 26; displaying the positive predictive value data point on the polymerase chain reaction test report 28.

The polymerase chain reaction test report 28 may remain in electronic form or be converted to another medium. The steps of the second reporting procedure may be performed in a different sequence and this disclosure should not be construed as to limit the second reporting procedure to the order described.

The second preferred embodiment of the system for providing an early indicator of true positive probability of a microbiology test sample rfto improve treatment response time and accuracy may further comprise a first preferred embodiment of a compliance feedback loop comprising the steps of: entering a blood collector data point into the electronic recordkeeping system; associating the blood collector data point to the polymerase chain reaction test result data point; generating a compliance report comprising the blood collector data point and polymerase chain reaction test data point. The compliance feedback loop is used to identify personnel using the collection method. Comparison of the compliance report to data for polymerase chain reaction tests collected by other methods may be used to identify personnel not using the collection method and targeting identified personnel for corrections.

Referring to FIG 5, a third preferred embodiment for the means to identify a microbiology test sample is shown comprising a needle hub 29 having a luer connector 30, a luer needle 31, and a marking device 32. As illustrated in FIG 6, the marking device 32 contacts an area on a device for collecting a microbiology test sample 33 leaving a mark for tracking. The needle hub 30 may be constructed from injection molded thermoplastics and connect to the luer connector by screw threading. The luer needle may be constructed from injection molded thermoplastics, steel, and an elastomer. The luer needle may be beveled to allow for penetration of elastomeric seals. The marking device may be comprised of a filler material at least partially saturated with an oil based dye. The marking device may further comprise a cover material that reduces evaporation of the oil based dye and is removable by advancement of the device for collecting a microbiology test sample 33 into the needle hub 29 as shown in FIG 6. The needle hub 29, the luer connector 30, the luer needle 31, and the marking device 32 may be constructed from other materials and assembled in a variety of configurations and this disclosure should not be construed as to limit the third embodiment for a means to identify a microbiology test to those materials and configurations described.

The present invention may include steps, procedures, materials, devices, apparatuses, and other elements from any preferred embodiment described or anticipated embodiments in various combinations and this disclosure should not be construed as to limit the present invention to the preferred embodiments described.

## Claims

1. A system for providing an early indicator of true positive probability of a microbiology test sample to improve treatment response time and accuracy comprising the steps of: providing a means to collect a first body fluid sample comprising a first seal having a contact area and a first hollow body; providing a means to maintain sterility of the contact area; providing a means to identify a microbiology test sample; providing a means to collect a microbiology test sample comprising a second seal and a second hollow body; providing a means to access a patient's body fluid vessel comprising a first end and a second end; drawing a microbiology test sample using a collection method comprising the steps of collecting a body fluid sample by piercing through a patient's skin using said first end of said means to access a patient's body fluid vessel and piercing through said first seal using said second end of said means for accessing a patient's body fluid vessel such that potential contaminants are drawn into said means for collecting a first body fluid sample, collecting a microbiology test sample by piercing though said second seal of said means to collect a microbiology test sample using said second end of said means to access a patient's body fluid vessel; performing a microbiology test using said microbiology test sample to yield a test result; using said means to identify a microbiology test sample to classify said test result into a group of test results; calculating a contamination rate for said group of test results; generating an indicator of true positive probability based on said contamination rate; generating a report comprising said test result and said indicator of true positive probability.

2. A system for providing an early indicator of true positive probability of a microbiology test sample to improve treatment response time and accuracy according to claim 1 in which said means for identifying a microbiology test sample is attached to said means for collecting a first body fluid sample.

3. A system for providing an early indicator of true positive probability of a microbiology test sample to improve treatment response time and accuracy according to claim 1 or 2 in which said means for identifying a microbiology test sample is attached to said means for collecting a microbiology test sample.

4. A system for providing an early indicator of true positive probability of a microbiology test sample to improve treatment response time and accuracy according to any preceding claim in which said means for identifying a microbiology test sample is a scannable identifier.

5. A system for providing an early indicator of true positive probability of a microbiology test sample to improve treatment response time and accuracy according to any preceding claim in which said means for identifying a microbiology test sample is a needle holder having a marking device that may be coupled to said means to access a patient's body fluid vessel.

6. A system for providing an early indicator of true positive probability of a microbiology test sample to improve treatment response time and accuracy according to any preceding claim in which said microbiology test is a blood culture test.

7. A system for providing an early indicator of true positive probability of a microbiology test sample to improve treatment response time and accuracy according to any preceding claim in which said microbiology test is a polymerase chain reaction test.

8. A system for providing an early indicator of true positive probability of a microbiology test sample to improve treatment response time and accuracy according to any preceding claim in which said indicator of contamination probability is a positive predictive value.

9. A system for providing an early indicator of true positive probability of a microbiology test sample to improve treatment response time and accuracy comprising the steps of: collecting a microbiology test sample into a device using a collection method; attaching a scannable identifier onto said device; performing a microbiology test using said microbiology test sample to yield a microbiology test result; entering a microbiology test result data point that corresponds to said microbiology test result into an electronic recordkeeping system; entering an identifier data point indicating presence of said scannable identifier on said device into said electronic recordkeeping system; associating said identifier data point with said microbiology test result data point within said electronic recordkeeping system; calculating a contamination rate for a group of test results collected using said collection method; generating an indicator of true positive probability based on said contamination rate; generating an indicator of true positive probability data point corresponding to said indicator of true positive probability; generating a report comprising said microbiology test result data point and said indicator of true positive probability data point.

10. A system for providing an early indicator of true positive probability of a microbiology test sample to improve treatment response time and accuracy according to claim 9 in which said scannable identifier is a bar code.

11. A system for providing an early indicator of true positive probability of a microbiology test sample to improve treatment response time and accuracy according to claim 9 or 10 in which said scannable identifier is a radiofrequency tag.

12. A system for providing an early indicator of true positive probability of a microbiology test sample to improve treatment response time and accuracy according to any of claims 9 - 11 in which said device is a blood culture bottle.

13. A system for providing an early indicator of true positive probability of a microbiology test sample to improve treatment response time and accuracy according to any of claims 9 - 12 in which said indicator of true positive probability is a positive predictive value.
